# FASCICULE DE BREVET EUROPEEN

(11) **EP 0 825 883 B1**
(45) Date de publication et mention de la délivrance du brevet: **08.03.2000**
(21) Numéro de dépôt: 96914931.9
(22) Date de dépôt: 17.04.1996
(51) Int. Cl.: A61M 5/20

(54) **AUTO-INJECTEUR RECHARGEABLE**
NACHLADBARER AUTO-INJEKTOR
RELOADABLE AUTO-INJECTOR

(30) Priorité: 18.04.1995 FR 9504579
(43) Date de publication de la demande: 04.03.1998
(73) Titulaire: TEBRO S.A., 1118 Luxembourg (LU)
(72) Inventeur: STRADELLA, Guiseppe, I-16032 Camogli (IT)
(74) Mandataire: CAPRI SARL
(86) Numéro de dépôt international: EP9601603
(87) Numéro de publication internationale: WO9632974

(56) Documents cités:
- EP-A- 0 666 084
- WO-A-94/11041
- DE-C- 902 776
- FR-A- 2 654 938

## Description

La présente invention concerne un auto-injecteur rechargeable, c'est-à-dire un appareil permettant d'injecter automatiquement le contenu d'une seringue dans le corps d'un patient.

Dans le domaine pharmaceutique, des dispositifs automatiques d'injection ou auto-injecteurs ont été développés dans le but de faciliter la distribution de certains médicaments au moyen d'une seringue.

Un exemple d'un tel auto-injecteur est divulgué dans le document WO 94/11041. Cet appareil incorpore un premier dispositif de pénétration automatique de l'aiguille dans la corps du patient, et un second dispositif d'injection automatique du produit, ce second dispositif n'étant actionné qu'après que l'aiguille ait complètement pénétrée dans le corps du patient.

Un inconvénient de cet appareil, comme de la plupart des auto-injecteurs actuels, est que ce sont des systèmes jetables destinés à une utilisation unique.

Or, pour des raisons écologiques mais aussi économiques, il est apparu un besoin pour des auto-injecteurs rechargeables, permettant de réutiliser plusieurs fois le même appareil.

Divers dispositifs rechargeables ont été élaborés pour répondre à ce besoin mais ils présentent tous les particularités d'être relativement chers, assez compliqués à utiliser et de ne résoudre parfois que partiellement le problème du traitement des déchets et de la protection de l'environnement.

L'inconvénient principal lié à l'utilisation d'un tel auto-injecteur rechargeable réside dans sa manipulation complexe en comparaison de celle d'un auto-injecteur jetable. En effet du fait que tous les auto-injecteurs sont actionnés par un ressort comprimé, plusieurs actions sont nécessaires avant de pouvoir réutiliser l'appareil. Ainsi, il faut ouvrir l'appareil, réamorcer ou réarmer le ressort, remplacer la seringue vide par une nouvelle seringue puis refermer l'appareil. Ces étapes sont souvent assez complexes et sont généralement réalisées en dévissant la partie de l'auto-injecteur qui porte la seringue, en recomprimant le ressort au moyen d'un outil séparé ou à l'aide d'un curseur coulissant actionné par l'utilisateur et agissant sur ledit ressort, puis en revissant la partie de l'auto-injecteur portant la seringue, après avoir remplacée celle-ci. Dans certains dispositifs existants, c'est toute la partie de l'auto-injecteur portant la seringue qui est remplacée.

Les différentes étapes précitées pour réarmer les appareils existants sont souvent trop compliquées pour de nombreux utilisateurs et peuvent entraîner une mauvaise utilisation de l'auto-injecteur ce qui peut avoir des résultats potentiellement dangereux pour le patient ou réduire l'effet du traitement.

Il est connu du document DE-90 27 776 un appareil d'injection automatique rechargeable. Cet appareil comporte un dispositif de pénétration de l'aiguille qui comprend un ressort déplaçant d'abord l'aiguille à l'intérieur de l'appareil vers la peau du patient, et un dispositif d'injection du produit dans lequel le même ressort déplace ensuite un piston dans un réservoir renfermant le produit. L'appareil est actionné par un bouton-poussoir.

Cet appareil présente plusieurs inconvénients. Ainsi, il est compliqué et cher à fabriquer et à assembler en raison du nombre élevé de pièces constitutives. D'autre part, son dispositif de pénétration de l'aiguille implique le déplacement de celle-ci à l'intérieur de l'appareil sous l'effet dudit ressort. Cette mise en oeuvre est désavantageuse car elle fait dépendre l'efficacité de la pénétration de l'aiguille, qui doit être de préférence complète, de la raideur du ressort. Or, ce ressort doit encore être suffisamment tendu après la pénétration de l'aiguille pour assurer l'injection du produit. Le réarmement de ce ressort est donc assez difficile et fastidieux, particulièrement pour des personnes faibles. De plus, l'appareil étant actionné par un bouton-poussoir, il existe un risque d'actionnement accidentel, par exemple avant que l'appareil soit placé contre le corps du patient à l'endroit souhaité.

Il est donc apparu intéressant au demandeur de développer un auto-injecteur du type rechargeable qui ne présente pas les inconvénients précités, mais qui implique au contraire les avantages d'être peu coûteux à fabriquer et d'une manipulation extrêmement aisée, simple et fiable, tant pour l'actionnement que pour le réarmement.

La présente invention a donc pour objet un auto-injecteur rechargeable comprenant une partie de boîtier destinée à recevoir une seringue, et une partie de couvercle, ledit auto-injecteur incorporant un dispositif d'injection automatique du produit contenu dans la seringue, ledit dispositif d'injection comportant :
- un piston comportant, dans la position verticale de l'auto-injecteur avec la seringue en bas, une partie haute et une partie basse, ladite partie basse coopérant avec le piston de la seringue, ledit piston étant mobile, sous l'effet d'un ressort d'actionnement, entre une position armée et une position de fin de course, ledit ressort étant comprimé dans ladite position armée, et
- des moyens de déclenchement mobiles entre une position de blocage où ils maintiennent le piston dans sa position armée, et une position de libération du piston, lesdits moyens de déclenchement étant libérés de leur position de blocage par un organe d'actionnement,
l'auto-injecteur comportant en outre des moyens de réarmement du dispositif d'injection automatique, caractérisé en ce que lesdits moyens de réarmement comportent un organe d'armement coopérant avec au moins un élément solidaire du piston lors de l'opération de fermeture de ladite partie de couvercle pour ramener ledit piston de sa position de fin de course vers sa position armée, ledit piston coulissant dans un manchon fixe de la partie de couvercle qui comporte au moins une fente axiale à travers laquelle ledit au moins un élément solidaire du piston fait saillie, pour coopérer avec ledit organe d'armement.

L'auto-injecteur selon l'invention présente donc l'avantage d'éliminer l'étape de réarmer le ressort du dispositif d'injection automatique, ceci étant réalisé simultanément à la manipulation du couvercle de l'auto-injecteur.

De préférence, il est prévu un manchon de commande mobile entre une position de verrouillage, où il maintient les moyens de déclenchement dans leur position de blocage et donc le piston dans sa position armée, et une position de déverrouillage, où lesdits moyens de déclenchement viennent dans leur position de libération du piston, ledit manchon de commande étant sollicité vers sa position de verrouillage par un ressort et étant forcé dans sa position de déverrouillage par ledit organe d'actionnement.

Avantageusement, lesdits moyens de déclenchement sont élastiques et comportent un élément d'interaction coopérant sur son côté intérieur avec la partie haute du piston et sur son côté extérieur avec le manchon de commande, ledit élément d'interaction maintenant ledit manchon de commande dans sa position de déverrouillage lorsque lesdits moyens élastiques de déclenchement sont dans leur position de libération du piston, ladite partie haute du piston comportant une partie de diamètre réduit qui coopère avec ledit élément d'interaction lorsque le piston se trouve dans sa position armée, de sorte que lesdits moyens élastiques de déclenchement adoptent leur position de blocage dudit piston, libérant simultanément ledit manchon de commande qui adopte sa position de verrouillage en venant s'engager autour dudit élément d'interaction desdits moyens élastiques de déclenchement, empêchant ainsi ceux-ci de revenir à leur position de libération du piston, le piston étant alors bloqué dans sa position armée.

En particulier, la partie haute du piston comporte un cylindre tubulaire creux dont la surface extérieure coopère avec ledit élément d'interaction desdits moyens élastiques de déclenchement, ledit cylindre tubulaire comportant à son extrémité proximale par rapport à la seringue une partie tronconique formant la partie de diamètre réduit du piston, ledit cylindre tubulaire recevant une extrémité dudit ressort d'actionnement du piston, l'autre extrémité dudit ressort étant solidaire du boîtier de l'auto-injecteur, de sorte que lors de l'ouverture et/ou de la fermeture de la partie de couvercle de l'auto-injecteur, ledit cylindre tubulaire du piston coulisse à l'intérieur desdits moyens de déclenchement en entraînant le ressort, de sorte que le ressort se comprime, jusqu'à ce que la partie de diamètre réduit du piston coopère avec l'élément d'interaction pour bloquer le piston dans sa position armée.

Selon la première variante, lesdits moyens de déclenchement sont réalisés sous la forme d'au moins une patte élastique et ledit élément d'interaction est réalisé sous la forme d'un ergot disposé à l'extrémité libre de ladite patte élastique, ladite au moins une patte élastique étant amenée dans sa position de libération de piston dès lors que ledit manchon de commande est forcé dans sa position de déverrouillage et ladite au moins une patte élastique étant amenée dans sa position de blocage du piston sous l'effet de la force exercée par ledit manchon de commande, dès lors que ledit élément d'interaction coopère avec ladite partie de diamètre réduit dudit piston.

Selon une seconde variante, lesdits moyens de déclenchement comprennent un anneau fendu qui adopte sa position de libération du piston dès lors que le manchon de commande est forcé dans sa position de déverrouillage et qui adopte sa position de blocage du piston dès lors qu'il coopère avec la partie de diamètre réduit dudit piston.

Selon un mode de réalisation de l'invention, l'auto-injecteur comporte une partie de couvercle emboîtable sur la partie de boîtier, le dispositif d'injection automatique du produit contenu dans la seringue étant disposé dans la partie de couvercle, l'emboîtement de ladite partie de couvercle sur ladite partie de boîtier lors de sa fermeture amenant ledit piston dans sa position armée et lesdits moyens de déclenchement dans leur position de blocage.

De préférence, ladite partie de couvercle et ladite partie de boîtier sont de forme générale cylindrique, ladite partie de couvercle s'emboîtant axialement sur ladite partie de boîtier, ladite partie de boîtier comportant lesdits moyens de réarmement qui comprennent ledit organe d'armement coopérant avec ledit au moins un élément solidaire du piston lors dudit emboîtement axial de ladite partie de couvercle sur ladite partie de boîtier pour amener ledit piston dans sa position armée.

Avantageusement, ledit organe d'armement de la partie de boîtier s'emmanche autour dudit manchon fixe, lors de l'emboîtement de la partie de couvercle sur la partie de boîtier, en agissant sur ledit au moins un élément solidaire du piston pour amener ledit piston dans sa position armée.

De manière avantageuse, ledit organe d'armement est cylindrique et comporte au moins une fente axiale pour permettre audit au moins un élément solidaire du piston de coulisser par rapport audit organe d'armement lorsque le piston se déplace de sa position armée vers sa position de fin de course, ledit organe d'armement pouvant être déplacé en rotation autour dudit manchon fixe entre une position angulaire d'armement, où il coopère avec ledit au moins un élément solidaire du piston pour armer ledit piston, et une position angulaire de libération, où ladite au moins une fente axiale de l'organe d'armement est disposée vis-à-vis dudit au moins un élément solidaire du piston.

De préférence, ladite partie de boîtier comporte un tube pouvant coulisser axialement par rapport audit organe d'armement entre une position de repos où il recouvre l'aiguille de la seringue et une position d'actionnement où il agit comme organe d'actionnement, son extrémité opposée à la seringue libérant les moyens de déclenchement du dispositif d'injection, ledit tube comportant un moyen de blocage qui empêche tout déplacement axial du tube sur ledit organe d'armement lorsque celui-ci est dans sa position angulaire d'armement et qui permet ledit déplacement axial lorsque l'organe d'armement est dans sa position angulaire de libération.

Avantageusement, ledit moyen de blocage du tube est réalisé sous la forme d'un doigt de blocage élastique saillant à l'extérieur dudit tube, ledit tube comportant en outre un doigt de butée saillant à l'extérieur dudit tube qui ne permet une séparation de la partie de boîtier de la partie de couvercle que lorsque l'organe d'armement est dans sa position angulaire de libération.

En particulier, la partie de couvercle comporte une enveloppe externe cylindrique qui s'emboîte autour du tube de la partie de boîtier, le diamètre intérieur de ladite enveloppe externe étant environ identique au diamètre extérieur dudit tube, de sorte que l'emboîtement de ladite enveloppe sur ledit tube force ledit doigt de blocage élastique vers l'intérieur pour bloquer le déplacement axial du tube par rapport audit organe d'armement, l'enveloppe comportant à son extrémité ouverte une encoche d'introduction pour introduire ladite patte de blocage saillante, ladite encoche étant disposée circonférentiellement de telle sorte que l'organe d'armement est disposé dans sa position angulaire d'armement lors de l'emboîtement de la partie de couvercle sur la partie de boîtier, l'enveloppe comportant sur sa surface intérieure au moins une rainure axiale s'étendant jusqu'à ladite extrémité ouverte, ladite au moins une rainure étant décalée angulairement par rapport à ladite encoche de telle sorte que ladite patte de blocage pénètre dans ladite au moins une rainure axiale lorsque l'organe d'armement est dans sa position angulaire de libération.

De préférence, ledit organe d'armement comporte sur sa surface intérieure, à proximité de son extrémité qui coopère avec ledit au moins un élément solidaire du piston, une rainure circonférentielle de solidarisation qui s'encliquète de manière amovible, après l'armement du piston, sur une nervure circonférentielle complémentaire prévue sur le manchon fixe, lesdites nervures et rainures de solidarisation assurant une solidarisation amovible de la partie de boîtier avec la partie de couvercle avant et après l'actionnement de l'auto-injecteur, et assurant une fixation inamovible de la partie de boîtier sur la partie de couvercle pendant l'actionnement de l'auto-injecteur, l'extrémité du tube bloquant ladite rainure sur ladite nervure lorsque ledit tube est dans sa position d'actionnement.

Avantageusement, il est prévu un organe élastique sollicitant ladite seringue légèrement hors de la partie de boîtier de l'auto-injecteur lorsque la partie de couvercle est retirée ou ouverte, facilitant ainsi la préhension de ladite seringue. Cette mise en oeuvre permet au patient de remplacer très simplement la seringue usagée par une nouvelle seringue, sans risquer de se blesser.

L'auto-injecteur rechargeable selon ce mode de réalisation présente notamment les avantages suivants :
- son utilisation et son rechargement sont extrêmement simples,
- il ne nécessite qu'un nombre minimal de mouvements de la part de l'utilisateur pour le recharger après une utilisation précédente : ouverture par traction axiale, changement de la seringue ou de toute la partie de boîtier, emboîtement axial, rotation,
- il élimine tout risque de déclenchement non souhaité ou partiel,
- il élimine toute possibilité d'erreur de manipulation,
- il élimine tout risque de blessure de l'utilisateur.

D'autres caractéristiques et avantages de la présente invention apparaîtront au cours de la description détaillée suivante de deux modes de réalisation particuliers de l'invention donnés à titre d'exemples non limitatifs, en regard des dessins joints.

Sur les dessins :
- la figure 1 représente une vue en coupe verticale partielle de l'auto-injecteur selon un mode de réalisation préféré de l'invention, après réarmement du dispositif d'injection automatique,
- la figure 2 représente une vue similaire à celle de la figure 1, avant actionnement du dispositif d'injection automatique,
- la figure 3 représente une vue en coupe verticale d'une variante de l'auto-injecteur des figures 1 et 2, après actionnement du dispositif d'injection automatique,
- les figures 4 et 5 représentent des vues agrandies de la partie supérieure, respectivement des figures 1 et 2,
- la figure 6 représente une vue similaire à celle de la figure 4, la partie de couvercle étant tournée à 90° pour montrer la coopération de l'organe d'armement avec le piston du dispositif d'injection,
- la figure 7a représente une vue de détail de face du doigt de blocage selon le premier mode de réalisation de l'invention,
- les figures 7b et 7c représentent des vues partielles en coupe verticale du doigt de blocage de la figure 6, respectivement avant et après actionnement de l'auto-injecteur,
- la figure 8 est une vue de détail de face du doigt de butée selon le premier mode de réalisation de l'invention,
- les figures 9a et 9b représentent des vues partielles en coupe verticale du doigt de butée de la figure 8, respectivement avant et après actionnement de l'auto-injecteur,
- la figure 10a est une vue de dessous en coupe horizontale selon la ligne I-I de la figure 4, dans la position angulaire d'armement de l'organe d'armement,
- la figure 10b est une vue de dessous en coupe horizontale selon la ligne II-II de la figure 5, dans la position angulaire de libération de l'organe d'armement,
- la figure 11 représente une vue schématique en coupe verticale d'une partie d'un auto-injecteur selon un mode de réalisation qui n'est pas couvert par la présente invention, le couvercle étant partiellement ouvert,
- la figure 12 représente une vue en coupe verticale d'une variante de l'auto-injecteur de la figure 11, le couvercle, fermé, n'étant que partiellement représenté pour montrer la structure interne, et
- les figures 13 et 14 représentent des vues en coupe verticale du dispositif d'injection automatique des figures 11 et 12 et de ses moyens de réarmement respectivement avant et après le réarmement de l'auto-injecteur.

L'invention concerne un auto-injecteur rechargeable. Par rechargeable, on entend que le même auto-injecteur, c'est-à-dire un appareil incorporant un dispositif d'injection automatique, peut être utilisé plusieurs fois avec des seringues différentes qui sont généralement du type "prérempli", c'est-à-dire prêtes à l'emploi.

En référence aux figures, l'auto-injecteur selon l'invention comporte une seringue 3 qui est reçu dans la partie de boîtier de telle manière à être fixement positionnée. Avantageusement, on peut prévoir un organe élastique (non représenté) agissant sur la seringue 3, par exemple un ressort disposé sous ladite seringue dans la partie de boîtier 101 qui sollicite ladite seringue légèrement hors de la partie de boîtier lorsque la partie de couvercle 102 est ouverte ou retirée. Ainsi, après son utilisation, la seringue vide fait légèrement saillie hors de son logement dans la partie de boîtier lorsque la partie de couvercle est ouverte ou retirée, de sorte qu'il est très facile de la saisir pour la remplacer.

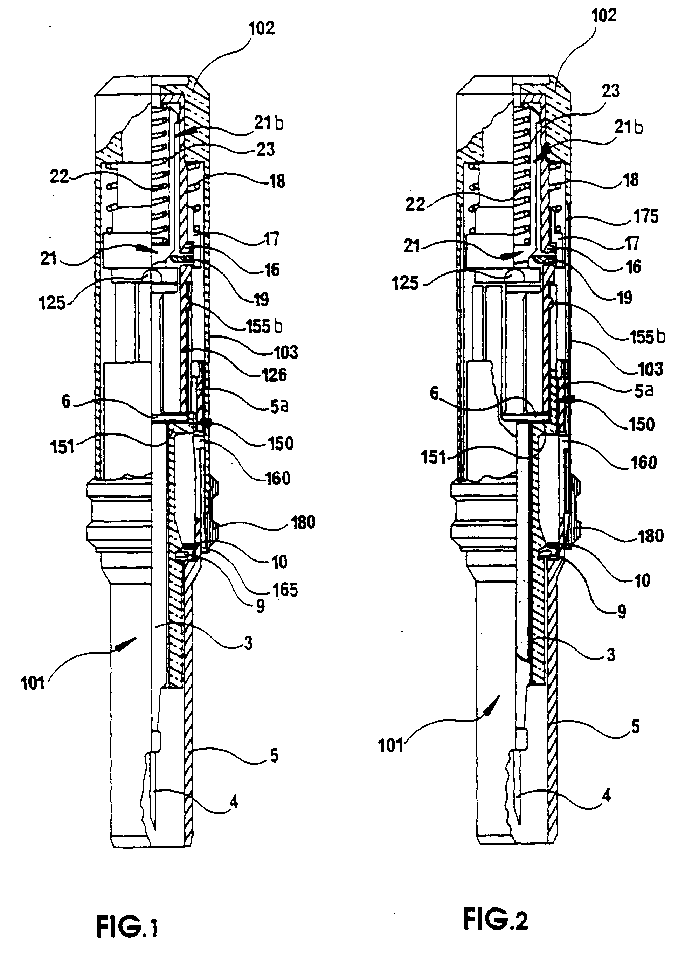

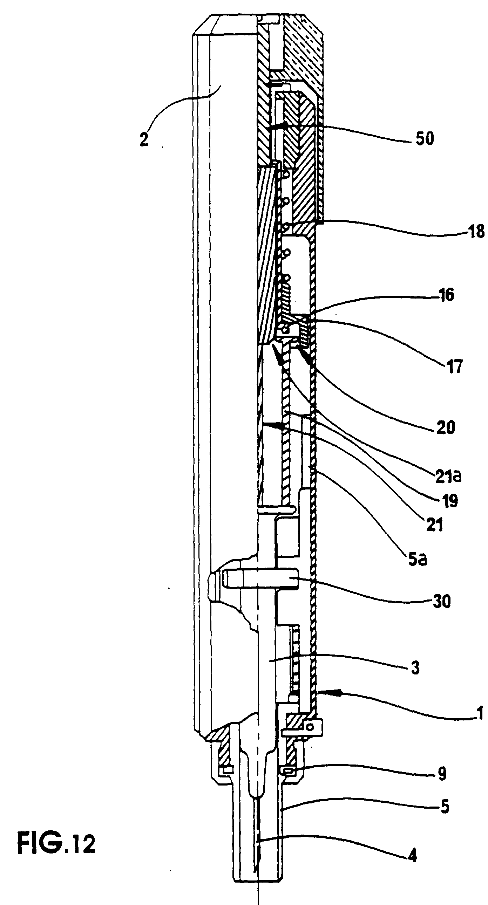

Le dispositif d'injection automatique du produit contenu dans la seringue correspond sensiblement à celui décrit dans le document WO 94/11041. Il comporte un piston 21 coopérant avec le piston de la seringue (non représenté), ledit piston 21 étant mobile, sous l'effet d'un ressort 22, entre une position armée, où ledit ressort 22 est comprimé, et une position de fin de course. Le piston 21 est maintenu dans sa position armée par des moyens de déclenchement 19 qui sont de préférence élastiques et qui comportent avantageusement un élément d'interaction 20. Par exemple, ils peuvent être réalisés sous la forme d'au moins une patte élastique 19 comportant à son extrémité libre un ergot 20. Dans ce cas, on prévoit de préférence deux pattes élastiques 19 diamétralement opposées par rapport au piston (voir figures 12, 13 et 14). De manière alternative, lesdits moyens de déclenchement peuvent également être réalisés sous la forme d'un anneau fendu (voir figures 1 à 5).

Lesdits moyens de déclenchement 19 sont eux-même mobiles entre une position de blocage, où ils maintiennent le piston 21 dans sa position armée, et une position de libération dudit piston 21.

Il est en outre prévu un manchon de commande 17 qui est mobile entre une position de verrouillage, où il maintient les moyens de déclenchement 19 dans leur position de blocage et donc le piston 21 dans sa position armée, et une position de déverrouillage, où ledit manchon de commande 17 ne bloque plus lesdits moyens de déclenchement 19 et où ceux-ci sont amenés dans leur position de libération du piston. Ce manchon de commande 17 est sollicité dans sa position de verrouillage par un ressort 18 et est forcé dans sa position de déverrouillage par un organe d'actionnement. Cet organe d'actionnement peut être quelconque et peut soit être actionné directement par l'utilisateur, soit faire partie d'un dispositif de pénétration automatique de l'aiguille tel que décrit dans le document WO 94/11041 et tel que représenté plus précisément sur les figures 1 à 3 et 12. Dans ce cas, il est prévu un tube coulissant 5 entourant l'aiguille 4 de la seringue 3, ce tube 5 étant appliqué sur la peau du patient et une force suffisante étant nécessaire pour permettre audit tube 5 de coulisser, celui-ci étant d'abord retenu par un organe tel qu'un anneau fendu 9 de sorte que lors de la pression de l'auto-injecteur sur la peau, une certaine quantité d'énergie est préalablement emmagasinée jusqu'à ce que ledit anneau 9 s'écarte sous l'effet de ladite force, ledit tube 5 pouvant alors coulisser par rapport à l'aiguille de la seringue permettant à celle-ci de pénétrer dans le corps du patient, la pénétration complète de l'aiguille étant assurée par ladite énergie préalablement emmagasinée dans le tube 5. Dans cette mise en oeuvre, c'est l'extrémité 5a du tube 5 qui vient buter en fin de course contre le manchon de commande 17 et qui déclenche alors l'injection automatique du produit contenu dans la seringue. Ainsi, il est garanti que le produit n'est injecté qu'après que l'aiguille ait entièrement pénétrée dans le corps du patient.

Le piston 21 comporte une partie haute 21b et une partie basse 21c. La partie basse 21c, sensiblement en forme de tige, coopère avec le piston de la seringue pour injecter le produit contenu dans la seringue dans le corps du patient, lorsque le piston 21 se déplace de sa position armée vers sa position de fin de course sous l'effet du ressort d'actionnement 22.

La partie haute 21b du piston 21 coopère avec lesdits moyes de déclenchement 19. Comme représenté sur les figures 1 à 5, les moyens de déclenchement peuvent être réalisés sous la forme d'un anneau élastique fendu 19. Cet anneau 19 coopère d'une part avec la partie haute 21b du piston 21 sur son côté intérieur 20a et d'autre part avec le manchon de commande 17 sur son côté extérieur 20b. La partie haute 21b du piston 21 comporte une partie de diamètre réduit 21a qui coopère avec ledit anneau 19 lorsque le piston 21 se trouve dans sa position armée. Dans cette position, le manchon de commande 17, sollicité par son ressort 18, vient buter contre un épaulement 16 du boîtier, et se trouve alors dans sa position de verrouillage où il maintient ledit anneau 19 dans sa position de blocage. L'anneau 19 est donc soumis à la force exercée par le ressort 22 sur le piston 21. Lorsque l'organe d'actionnement vient forcer le manchon de commande 17 dans sa position de déverrouillage, l'anneau fendu 19 est libéré sur son côté extérieur 20b et il s'écarte vers l'extérieur au niveau de sa fente sous l'effet de ladite force exercée par le ressort 22 et/ou de son élasticité propre.

Le piston 21 est alors libéré et le produit contenu dans la seringue est injecté au patient. L'anneau fendu 19 est alors dans sa position de libération du piston où, par son côté extérieur 20b, il maintient le manchon de commande 17 dans sa position de déverrouillage. Ledit anneau fendu 19 est donc soumis à la force exercée par le ressort 18 sur le manchon de commande 17. Lorsque le piston 21 revient vers sa position armée et que l'anneau fendu 19 se retrouve en face de la partie de diamètre réduit 2 la du piston 21, il se referme vers l'intérieur sous l'effet de ladite force exercée par le ressort 18 et/ou de son élasticité propre.

Avantageusement, la partie haute 21b du piston 21 comporte un cylindre tubulaire creux 23 dont la surface extérieure coopère avec le côté intérieur 20a dudit anneau 19. Ce cylindre 23 reçoit une extrémité du ressort d'actionnement 22 et comporte à son extrémité proximale par rapport à la seringue, c'est-à-dire à son extrémité inférieure sur les figures, une partie tronconique formant ladite partie de diamètre réduit 21 a du piston 21.

Cette mise en oeuvre tronconique de la partie de diamètre réduit 2 la du piston 21 assure un glissement progressif dudit anneau 19 sur ladite partie de diamètre réduit 21a lorsqu'il vient dans ou quitte sa position de blocage et évite ainsi tout risque "d'auto-blocage" dudit anneau 19 dans sa position de blocage.

De même, le manchon de commande 17 peut comporter de manière similaire une partie tronconique dans sa partie qui coopère avec ledit anneau 19 pour éviter tout risque "d'auto-blocage" de celui-ci dans sa position de libération du piston 21.

Selon un mode de réalisation préféré représenté sur les figures 1 à 10b, l'auto-injecteur comporte une partie de boîtier 101 et une partie de couvercle 102 qui peut être désolidarisée de ladite partie de boîtier lors du changement de seringue. Ces deux parties constitutives de l'auto-injecteur sont cylindriques avec une direction de ce cylindre qui est circulaire pour permettre une rotation de la partie de couvercle par rapport à la partie de boîtier, comme cela sera expliqué plus en détail lors de la description du fonctionnement de ce premier mode de réalisation de l'invention. La partie de boîtier renferme la seringue, alors que la partie de couvercle comporte le dispositif d'injection automatique du produit contenu dans ladite seringue.

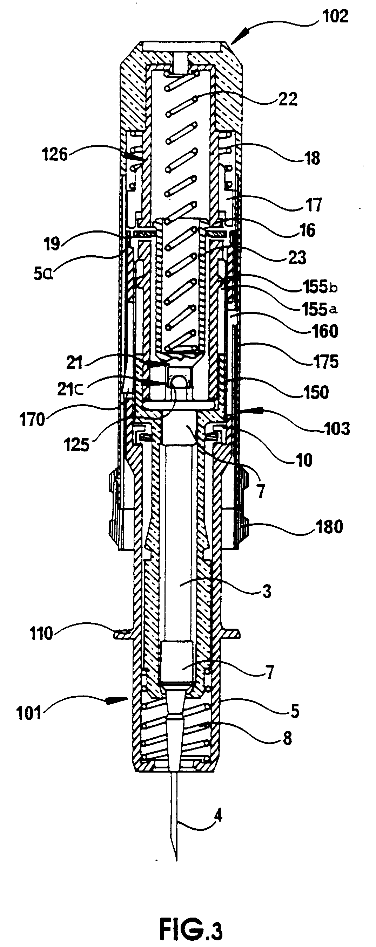

Selon ce mode de réalisation de l'invention, la partie de boîtier 101 reçoit et maintient fixement la seringue 3. Avantageusement, la seringue 3 est reçue dans une pièce environ cylindrique ci-après désignée par organe d'armement 150, dont la fonction de réarmement du dispositif d'injection de l'appareil sera décrite ultérieurement. Dans une première version, représentée notamment sur les figures 1 et 2, la seringue 3 est maintenue dans l'organe d'armement 150 au moyen d'une collerette 6 en appui sur un épaulement 151 dudit organe 150. Dans une autre version, représentée sur la figure 3, la seringue 3 comporte en outre une ou plusieurs parties de plus grand diamètre 7 qui s'emmanchent à l'intérieur dudit organe d'armement 150.

La partie de boîtier 101 comporte en outre un tube externe creux 5 disposé autour dudit organe d'armement 150. Ledit tube 5 est déplaçable axialement par rapport audit organe d'armement 150 entre une position de repos, où il recouvre l'aiguille 4 de la seringue 3, et une position d'actionnement, où il découvre ladite aiguille, lui permettant de pénétrer dans le corps du patient. Par contre, le tube 5 ne peut pas être déplacé en rotation par rapport à l'organe d'armement 150, de sorte qu'une rotation de la partie de boîtier 101, c'est-à-dire du tube 5, entraîne une rotation identique de l'organe d'armement 150.

Avantageusement, on prévoit entre le tube 5 et l'organe d'armement 150 un dispositif tel qu'un anneau fendu 9 dans une rainure pour maintenir le tube 5 dans sa position de repos, une force minimale devant être appliquée sur ledit tube pour faire sortir l'anneau fendu de la rainure et ainsi permettre au tube 5 de se déplacer en direction de sa position d'actionnement.

On assure ainsi que le tube 5 se déplace jusqu'à sa position d'actionnement et donc que l'aiguille pénètre entièrement dans le corps du patient. Lorsque le tube 5 est dans sa position d'actionnement, son extrémité 5a agit sur le manchon 17 et libère ainsi le dispositif d'injection de l'appareil, tel que décrit précédemment. Après le fonctionnement de l'auto-injecteur, le tube 5 est ramené vers sa position de repos pour d'une part recouvrir l'aiguille de la seringue et, pour d'autre part, permettre une prochaine utilisation de l'auto-injecteur. Avantageusement, comme visible sur la figure 3, on prévoit un ressort de retour 8 pour ramener automatiquement le tube 5 dans sa position de repos après l'utilisation de l'auto-injecteur. Ceci permet notamment d'éviter les risques de blessures. Pour ramener l'anneau fendu 9 dans sa rainure lors du retour du tube 5 dans sa position de repos, on prévoit avantageusement un organe de ramenée 10 solidaire dudit tube 5. Ainsi, le tube 5 est à nouveau prêt pour une prochaine utilisation de l'auto-injecteur.

Comme représenté sur les figures 1 à 7, le tube 5 comporte un moyen élastique de blocage 160, qui peut être réalisé sous la forme d'un doigt élastique 160 mobile entre une position de blocage, où ledit doigt est contraint vers l'intérieur et empêche le déplacement axial du tube 5 par rapport à l'organe d'armement 150, et une position de déblocage, où ledit doigt est saillant vers l'extérieur du tube 5 et ne bloque plus le déplacement axial dudit tube 5 sur ledit organe d'armement 150. La position de blocage du doigt 160 est représentée sur les figures 1, 4, 6 et 7b et la position de déblocage est représentée sur les figures 2, 3, 5 et 7c. Le tube 5 comporte en outre un moyen de butée 170, qui peut être réalisé sous la forme d'un doigt élastique de butée 170 saillant à l'extérieur dudit tube 5. La fonction de ces doigts de blocage 160 et de butée 170 sera décrite ultérieurement.

Avantageusement, lesdits doigts de blocage 160 et de butée 170 sont disposés diamétralement opposés sur ledit tube 5.

L'organe d'armement 150 comporte à son extrémité qui est opposée à l'aiguille 4 de la seringue 3, au moins une fente axiale 156. De préférence, l'organe d'armement 150 comporte deux fentes axiales 156 disposées diamétralement opposées par rapport à son axe central. Ces fentes axiales 156 sont destinées à permettre l'actionnement de l'auto-injecteur, comme cela sera décrit ultérieurement.

La partie de couvercle 102 de l'auto-injecteur selon le premier mode de réalisation de l'invention, comporte une enveloppe externe 103 qui est également cylindrique et est configurée de telle sorte à pouvoir s'emboîter sur la partie de boîtier 101, et plus particulièrement sur le tube 5. Le diamètre intérieur de l'enveloppe 103 est donc environ identique ou très légèrement supérieur au diamètre extérieur dudit tube 5, de sorte que l'emboîtement est réalisé sans jeu et sans frottement important.

L'enveloppe externe 103 de la partie de couvercle est munie, à son extrémité ouverte, d'une encoche 165, au niveau de laquelle le diamètre intérieur de ladite enveloppe 103 est augmenté. Avantageusement, cette encoche 165 a environ la même largeur que ledit doigt de blocage 160 de la partie de boîtier 101. De même, il est préféré qu'en direction axiale de l'enveloppe externe 103 en partant de l'extrémité ouverte, le diamètre de ladite encoche diminue progressivement jusqu'à ce qu'il égale le diamètre de l'enveloppe 103 elle-même.

L'enveloppe externe 103 comporte en outre sur sa surface intérieure au moins une rainure 175 s'étendant axialement sur une partie de la longueur de ladite enveloppe 103, à partir de son extrémité ouverte. Cette au moins une rainure axiale 175 est destinée à recevoir ledit doigt de blocage de la partie de boîtier 101 pour permettre d'actionner l'auto-injecteur. Avantageusement, l'enveloppe externe 103 comporte deux rainures axiales 175 identiques diamétralement opposées.

Selon l'invention, cette au moins une rainure axiale 175 est décalée circonférentiellement par rapport à ladite encoche 165, de préférence d'environ 90°.

La partie de couvercle 102 comporte à l'intérieur de ladite enveloppe 103 un manchon fixe creux 126 à l'intérieur duquel est disposé le piston 21 et qui comporte également l'anneau fendu 19 du dispositif d'injection automatique. Le piston 21 se déplace donc axialement à l'intérieur dudit manchon fixe 126 entre sa position armée et sa position de fin de course.

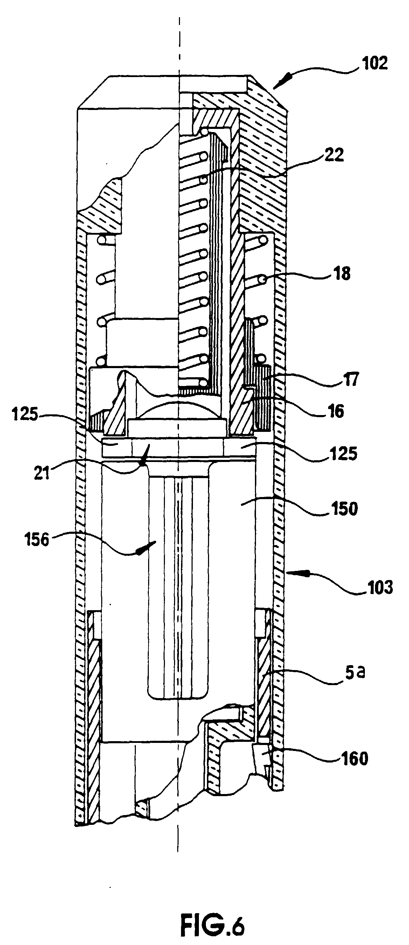

Selon ce mode de réalisation de l'invention, le piston 21 comporte dans sa partie basse 21c au moins un élément 125 solidaire du piston 21, réalisé par exemple sous la forme d'une ailette. De préférence, on prévoit deux ailettes 125 diamétralement opposées, comme représenté en particulier sur les figures 6, 10a et lOb. Ces ailettes 125 font saillie à l'extérieur du manchon fixe à travers deux fentes axiales 127 respectives dudit manchon fixe 126. Lors du déplacement du piston 21 entre ses positions armée et fin de course, lesdites ailettes 125 se déplacent donc en conséquence dans lesdites fentes axiales 127.

Le fonctionnement de l'auto-injecteur selon ce mode de réalisation préféré est le suivant.

L'utilisateur de l'auto-injecteur introduit une seringue 3 dans la partie de boîtier 101. Eventuellement, comme représenté sur la figure 3, la seringue 3 peut être inséparable de la partie de boîtier 101 auquel cas l'utilisateur est dispensé de l'étape de chargement de la seringue.

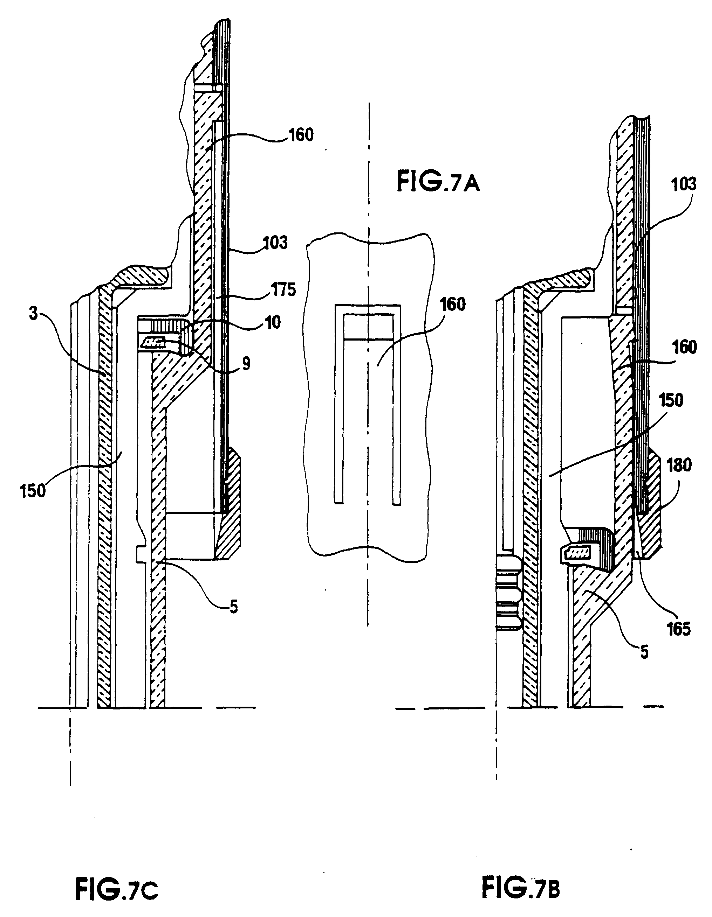

La partie de couvercle 102 comportant le dispositif d'injection est ensuite emboîtée sur la partie de boîtier 101. En raison du doigt de blocage 160 saillant à l'extérieur du tube 5 de la partie de boîtier 101, cet emboîtement n'est réalisable qu'en introduisant ledit doigt de blocage 160 dans ladite encoche 165 de la partie de couvercle 102. La diminution progressive du diamètre au niveau de l'encoche 165 force ledit doigt de blocage élastique 160 vers l'intérieur dans sa position de blocage, comme visible en particulier sur la figure 7b. Dans cette position, le doigt de blocage 160 se place sous un épaulement de l'organe d'armement 150, empêchant ainsi le déplacement axial dudit tube 5 sur ledit organe d'armement 150.

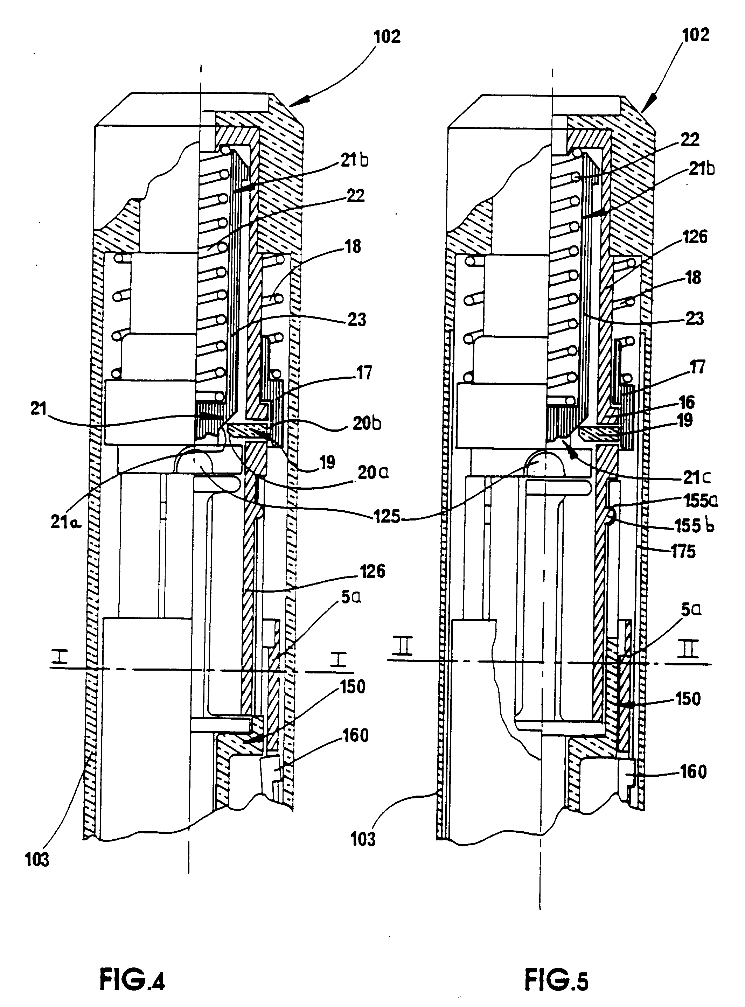

L'encoche 165 est disposée circonférentiellement de elle sorte que, comme visible sur la figure 6, lorsque la partie de couvercle 102 est emboîtée sur la partie de boîtier 101, l'extrémité supérieure de l'organe d'armement 150 appuie sur lesdites ailettes 125 du piston 21. Ainsi la continuation de l'emboîtement de la partie de couvercle sur la partie de boîtier déplace le piston 21 en direction de sa position armée dans laquelle le dispositif d'injection est armé, comme décrit précédemment. Cette position armée du piston 21 est représentée sur les figures 1 et 4. Dans cette position, le dispositif d'injection ne peut pas être actionné en raison du doigt de blocage 160 qui est dans sa position de blocage.

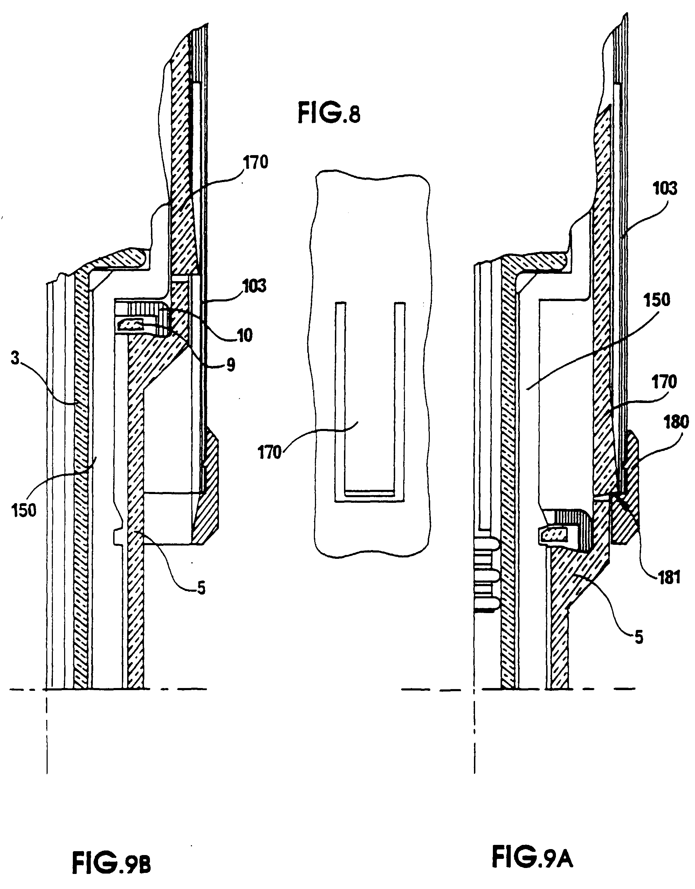

Pendant l'emboîtement, le doigt de butée 170 est également forcé vers l'intérieur pour passer par dessus puis venir s'appuyer sur un épaulement 181 de l'enveloppe 103 et ainsi empêcher une désolidarisation de la partie de boîtier 101 de la partie de couvercle 102. Cette position de butée est représentée sur la figure 9a.

Eventuellement, l'enveloppe 103 comporte à son extrémité ouverte une pièce rapportée 180 qui incorpore l'encoche 165 et l'épaulement 181, et qui comporte également des prolongements desdites rainures axiales 175 de l'enveloppe 103.

Lorsque la partie de boîtier 101 est emboîtée dans la partie de couvercle 102, l'organe d'armement 150 s'emboîte donc autour du manchon fixe 126. Comme visible en particulier sur les figures 4 et 5, le manchon fixe 126 comporte une nervure circonférentielle 155b sur laquelle s'encliquète une rainure correspondante 155a de l'organe d'armement 150. Cet encliquetage est réalisable du fait que l'organe d'armement comporte au moins une fente axiale 156, de préférence deux, de sorte que cet encliquetage est amovible relativement aisément et n'assure qu'une solidarisation minimale de la partie de boîtier 101 sur la partie de couvercle 102 avant et après actionnement de l'auto-injecteur. Eventuellement, pour faciliter encore davantage cet encliquetage, on peut prévoir une ou plusieurs fentes axiales supplémentaires de faible largeur dans l'organe d'armement 150. De même, la nervure 155h et sa rainure correspondante 155a présentent avantageusement un profil arrondi facilitant l'encliquetage et la séparation.

Pour actionner l'auto-injecteur, il est nécessaire de tourner la partie de boîtier 101 par rapport à la partie de couvercle 102, amenant ainsi l'organe d'armement 150 de sa position angulaire d'armement dans sa position angulaire de libération. Avantageusement, si l'enveloppe 103 comporte deux rainures axiales 175 diamétralement opposées décalées de 90° par rapport à l'encoche 165, la rotation peut se faire dans les deux sens d'un angle d'environ 90°.

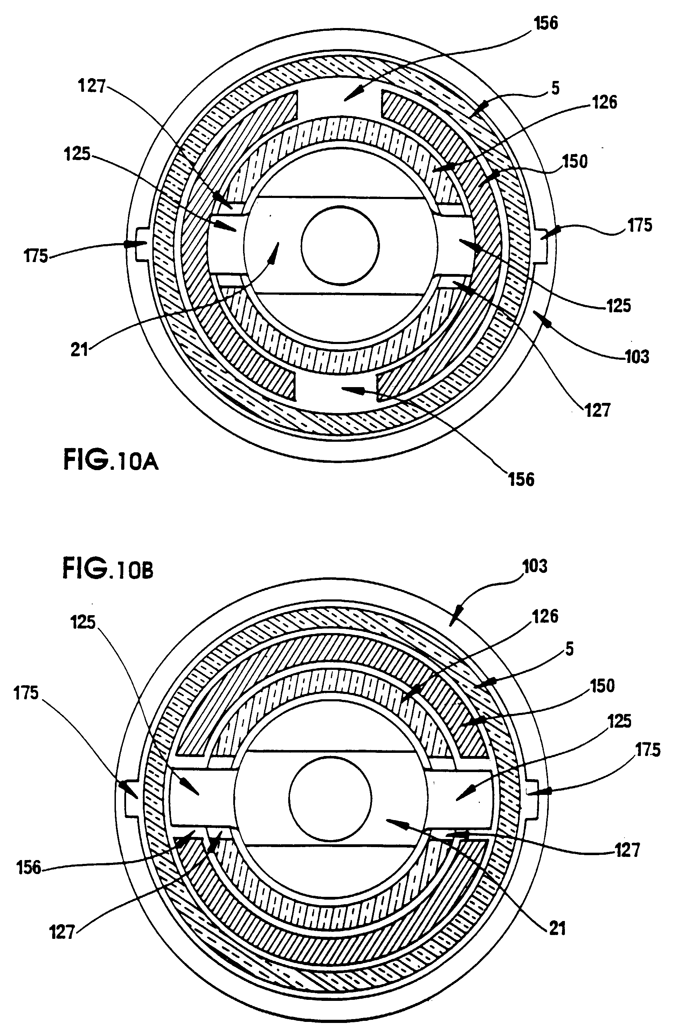

Ceci est représenté notamment sur les figures 10a et 10b, qui montrent des vues de dessous de coupes transversales de l'auto-injecteur selon les lignes I-I et II-II des figures 4 et 5.

Dans la position angulaire de libération de l'organe d'armement 150 (figure 10b), les ailettes 125 du piston 21 sont disposées en face des deux fentes axiales 156 de l'organe d'armement 150. Cette position est également représentée sur les figures 2 et 5, sur lesquelles il est visible que la patte de blocage 160 a pénétré dans l'une desdites deux rainures axiales 175 de l'enveloppe externe 103. Par conséquent, le déplacement axial du tube 5 sur l'organe d'armement 150 n'est plus empêché et l'auto-injecteur peut être actionné en l'appliquant sur la zone à injecter du corps. Lorsque la pression est suffisante, le tube 5 coulisse sur l'organe d'armement 150 et l'aiguille est découverte, pénétrant ainsi dans le corps du patient. Lorsque le tube 5 parvient à sa position d'actionnement, son extrémité 5a agit sur les moyens de déclenchement du dispositif d'injection, tels que décrit précédemment. Le piston 21 est donc libéré et il se déplace dans le manchon fixe 126 en direction de sa position de fin de course, ses deux ailettes 125 coulissant dans les deux fentes axiales 127 du manchon fixe 126 et dans les deux fentes axiales 156 de l'organe d'armement 150.

Lorsque l'enveloppe 103 comporte deux rainures axiales 175 diamétralement opposées et lorsque les doigts de blocage 160 et de butée 170 sont disposés diamétralement opposés sur le tube 5, le doigt de butée 170 pénètre également dans une desdites rainures axiales 175 lorsque l'organe d'armement 150 est dans sa position angulaire de libération. La partie de couvercle 102 n'est alors solidarisée à la partie de boîtier 101 que par l'intermédiaire de la rainure 155a et de la nervure 155b de solidarisation.

Lorsque l'auto-injecteur est actionné, le tube 5 coulisse sur l'organe d'actionnement 150, de sorte qu'au moment où l'extrémité 5a du tube 5 déclenche le dispositif d'injection, la partie du tube 5 située à proximité de l'extrémité 5a entoure la partie de l'organe d'armement 150 qui comporte ladite rainure 155a. Ainsi, pendant le fonctionnement du dispositif d'injection, la rainure 155a ne peut pas se désencliqueter de la nervure 155b, et la partie de couvercle 102 est donc fixement maintenue sur la partie de boîtier 101. Le produit contenu dans la seringue 3 est alors injecté automatiquement dans le corps du patient.

La position de fin de course du piston 21 est représentée sur la figure 3. L'aiguille 4 de l'auto-injecteur est alors retirée du corps du patient. De préférence, le tube 5 comporte un ressort de rappel 8 qui ramène le tube 5 dans sa position de repos où il entoure l'aiguille 4. Simultanément, la solidarisation entre l'organe d'armement 150 et le manchon fixe 126, par l'intermédiaire de la rainure 155a et de la nervure 155b, redevient amovible. La partie de boîtier 101 peut donc être séparée de la partie de couvercle 102 par simple traction, le doigt de butée 170 coulissant dans une des rainures axiales 175 de l'enveloppe 103, comme visible notamment sur la figure 9b.

Lorsque la partie de boîtier 101 est séparée de la partie de couvercle 102, soit on remplace uniquement la seringue 3 dans la partie de boîtier 101 et on réarme l'auto-injecteur selon le processus décrit ci-dessus, soit on remplace toute la partie de boîtier contenant la seringue vide par une autre partie de boîtier contenant une nouvelle seringue. Cette seconde variante peut s'avérer intéressante lorsqu'il n'est pas souhaitable que l'utilisateur puisse utiliser d'autres seringues que celles qui lui sont destinées.

Dans une variante de ce premier mode de réalisation de l'invention, on peut prévoir une fenêtre dans la partie de boîtier 101, permettant de voir l'intérieur de celle-ci pour contrôler la présence ou l'absence de seringue. D'autre part, comme représenté sur la figure 3, on peut avantageusement prévoir un épaulement 110 saillant radialement vers l'extérieur de la partie de boîtier 101 pour faciliter les manipulations lors de l'ouverture et de la fermeture et donc de l'armement de l'auto-injecteur.

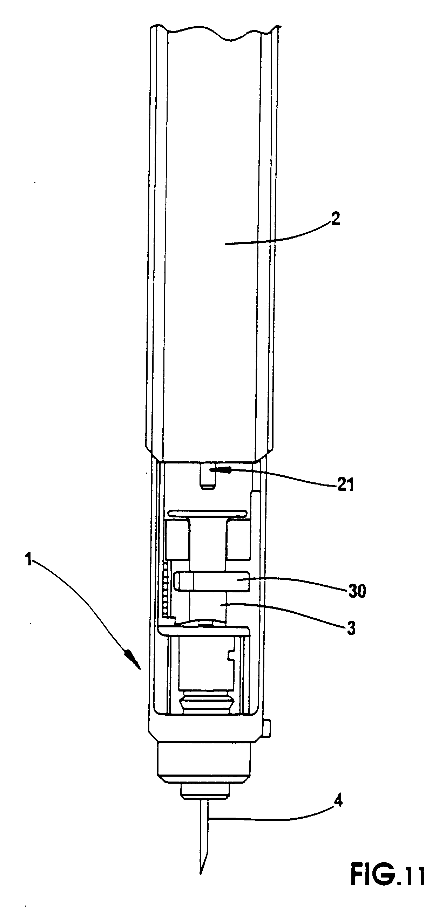

Sur les figures 11 à 14, il est représenté un mode de réalisation d'un auto-injecteur qui n'est pas couvert par l'invention, mais qui comporte une variante de réalisation des moyens de déclenchement.

Selon ce mode de réalisation, l'auto-injecteur comporte un boîtier 1 sensiblement cylindrique, la directrice de ce cylindre pouvant être quelconque, par exemple circulaire ou environ rectangulaire. Le boîtier 1 comporte un couvercle 2 coulissant sur ledit boîtier entre une position fermée et une position ouverte dans laquelle le boîtier 1 et le couvercle 2 restent solidaires l'un de l'autre. A l'intérieur du boîtier 1 sont disposés la seringue 3, le dispositif d'injection automatique du produit contenu dans ladite seringue et des moyens de réarmement dudit dispositif d'injection.

A la différence du premier mode de réalisation décrit précédemment, l'auto-injecteur ne se sépare plus en deux parties distinctes, c'est-à-dire que la partie de boîtier et la partie de couvercle restent en permanence liées l'une à l'autre.

Le dispositif d'injection automatique est identique à celui décrit précédemment, les mêmes références numériques désignant les mêmes éléments du dispositif. Il ne sera donc décrit ici qu'en ce qui concerne la variante de réalisation des moyens de déclenchement représentée sur les figures 12 à 14. Il est à noter que cette variante s'adapte également au mode de réalisation préféré précité.

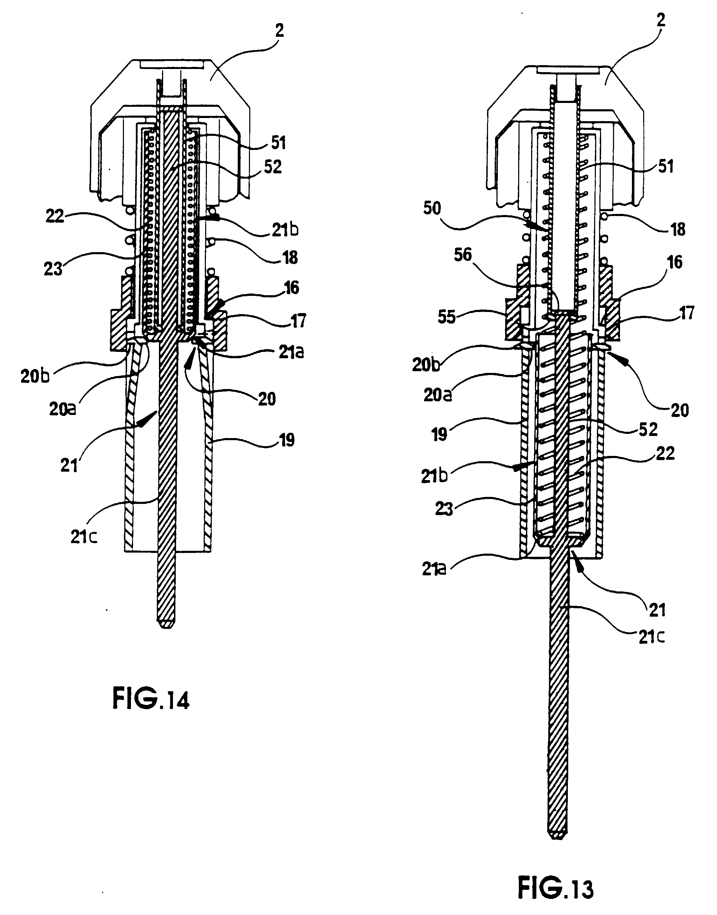

Ainsi, comme représenté sur les figures 12 et 14, ces moyens de déclenchement peuvent être réalisés sous la forme d'au moins une patte élastique pouvant s'infléchir soir vers l'intérieur, soit vers l'extérieur et comportant à son extrémité libre un ergot 20 agissant comme élément d'interaction. Avantageusement, on prévoit dans ce cas deux pattes élastiques 19 identiques diamétralement opposées par rapport au piston 21. Dans un but de simplification, la mise en oeuvre et le fonctionnement de ces deux pattes ne sera décrit ci-après qu'en référence à une seule patte. L'ergot 20 de la patte 19 coopère d'une part avec la partie haute 21b du piston 21 sur son côté intérieur 20a et d'autre part avec le manchon de commande 17 sur son côté extérieur 20b. C'est la partie de diamètre réduit 21a qui coopère avec ledit ergot 20 lorsque le piston 21 se trouve dans sa position armée. Dans cette position, le manchon de commande 17, sollicité par son ressort 18, vient buter contre un épaulement 16 du boîtier, et se trouve alors dans sa position de verrouillage où il maintient ledit ergot 20 de ladite patte élastique 19 dans sa position de blocage. La patte élastique 19 est donc soumise à la force exercée par le ressort 22 sur le piston 21. Lorsque l'organe d'actionnement vient forcer le manchon de commande 17 dans sa position de déverrouillage, l'ergot 20 est libéré sur son côté extérieur 20b et la patte 19 s'écarte vers l'extérieur sous l'effet de ladite force exercée par le ressort 22 et/ou de son élasticité propre, de manière similaire à l'anneau fendu du premier mode de réalisation.

Le piston 21 est alors libéré et le produit contenu dans la seringue est injecté au patient. La patte 19 est alors dans sa position de libération du piston où ledit ergot 20, sur son côté extérieur 20b, maintient le manchon de commande 17 dans sa position de déverrouillage. Ledit ergot 20 est donc soumis à la force exercée par le ressort 18 sur le manchon de commande 17. Lorsque le piston 21 revient vers sa position aimée et que l'ergot 20 se retrouve en face de la partie de diamètre réduit 21a du piston 21, la patte 19 s'écarte vers l'intérieur sous l'effet de ladite force exercée par le ressort 18 et/ou de son élasticité propre.

Comme représenté d'une part sur la figure 12 et d'autre part sur les figures 13 et 14, la patte 19 peut respectivement soit être fléchie vers l'extérieur quand elle est dans sa position de libération du piston 21 et non-fléchie quand elle est dans sa position de blocage du piston 21, soit être non-fléchie dans sa position de libération du piston 21 et fléchie vers l'intérieur dans sa position de blocage du piston 21.

Dans le premier cas, c'est principalement le piston 21 qui force la patte 19 dans sa position de libération une fois que le manchon de commande 17 est dans sa position de déverrouillage. Dans le deuxième cas, c'est principalement le manchon de commande 17 qui force la patte 19 dans sa position de blocage par l'intermédiaire de son ressort 18 une fois que l'ergot 20 se trouve face à la partie de diamètre réduit 21a du piston 21.

De manière identique au mode de réalisation préféré, la partie haute 21b du piston 21 comporte avantageusement un cylindre tuhulaire creux 23 dont la surface extérieure coopère avec le côté intérieur 20a dudit ergot 20. Ce cylindre 23 reçoit une extrémité du ressort d'actionnement 22 et comporte à son extrémité proximale par rapport à la seringue, c'est-à-dire à son extrémité inférieure sur les figures, une partie tronconique formant ladite partie de diamètre réduit 21a du piston 21.

Cette mise en oeuvre tronconique de la partie de diamètre réduit 2 la du piston 21 assure un glissement progressif dudit ergot 20 sur ladite partie de diamètre réduit 2 la lorsque la patte 19 vient dans ou quitte sa position de blocage et évite ainsi tout risque "d'auto-blocage" de ladite patte 19 dans sa position de blocage.

De même, le manchon de commande 17 peut comporter de manière similaire une partie tronconique dans sa partie qui coopère avec ledit ergot 20 pour éviter tout risque "d'auto-blocage" de ladite patte 19 dans sa position de libération du piston 21.

## Revendications

1. Auto-injecteur rechargeable comprenant une partie de boîtier (101) destinée à recevoir une seringue (3), et une partie de couvercle (102), ledit auto-injecteur incorporant un dispositif d'injection automatique du produit contenu dans la seringue, ledit dispositif d'injection comportant :
- un piston (21) comportant, dans la position verticale de l'auto-injecteur avec la seringue (3) en bas, une partie haute (21b) et une partie basse (21c), ladite partie basse (21c) coopérant avec le piston de la seringue, ledit piston (21) étant mobile, sous l'effet d'un ressort d'actionnement (22), entre une position armée et une position de fin de course, ledit ressort (22) étant comprimé dans ladite position armée, et
- des moyens de déclenchement (19) mobiles entre une position de blocage où ils maintiennent le piston (21) dans sa position armée, et une position de libération du piston (21), lesdits moyens de déclenchement (19) étant libérés de leur position de blocage par un organe d'actionnement,
l'auto-injecteur comportant en outre des moyens de réarmement du dispositif d'injection automatique, caractérisé en ce que lesdits moyens de réarmement comportent un organe d'armement (150) coopérant avec au moins un élément (125) solidaire du piston (21) lors de l'opération de fermeture de ladite partie de couvercle (102) pour ramener ledit piston (21) de sa position de fin de course vers sa position armée, ledit piston (21) coulissant dans un manchon fixe (126) de la partie de couvercle (102) qui comporte au moins une fente axiale (127) à travers laquelle ledit au moins un élément (125) solidaire du piston fait saillie, pour coopérer avec ledit organe d'armement (150).

2. Auto-injecteur selon la revendication 1, dans lequel est prévu un manchon de commande (17) mobile entre une position de verrouillage, où il maintient les moyens de déclenchement (19) dans leur position de blocage et donc le piston (21) dans sa position armée, et une position de déverrouillage, où lesdits moyens de déclenchement (19) viennent dans leur position de libération du piston (21), ledit manchon de commande (17) étant sollicité vers sa position de verrouillage par un ressort (18) et étant forcé dans sa position de déverrouillage par ledit organe d'actionnement.

3. Auto-injecteur selon la revendication 2, dans lequel lesdits moyens de déclenchement (19) sont élastiques et comportent un élément d'interaction (20) coopérant sur son côté intérieur (20a) avec la partie haute (21b) du piston (21) et sur son côté extérieur (20b) avec le manchon de commande (17), ledit élément d'interaction (20) maintenant ledit manchon de commande (17) dans sa position de déverrouillage lorsque lesdits moyens élastiques de déclenchement (19) sont dans leur position de libération du piston (21), ladite partie haute (21b) du piston (21) comportant une partie de diamètre réduit (2 la) qui coopère avec ledit élément d'interaction (20) lorsque le piston (21) se trouve dans sa position armée, de sorte que lesdits moyens élastiques de déclenchement (19) adoptent leur position de blocage dudit piston (21), libérant simultanément ledit manchon de commande (17) qui adopte sa position de verrouillage en venant s'engager autour dudit élément d'interaction (20) desdits moyens élastiques de déclenchement (19), empêchant ainsi ceux-ci de revenir à leur position de libération du piston (21), le piston (21) étant alors bloqué dans sa position armée.

4. Auto-injecteur selon la revendication 3, dans lequel la partie haute (21b) du piston (21) comporte un cylindre tubulaire creux (23) dont la surface extérieure coopère avec ledit élément d'interaction (20) desdits moyens élastiques de déclenchement (19), ledit cylindre tubulaire (23) comportant à son extrémité proximale par rapport à la seringue une partie tronconique formant la partie de diamètre réduit (21a) du piston (21), ledit cylindre tubulaire (23) recevant une extrémité dudit ressort (22) d'actionnement du piston (21), l'autre extrémité dudit ressort (22) étant solidaire du boîtier de l'auto-injecteur, de sorte que lors de l'ouverture et/ou de la fermeture de la partie de couvercle (2, 102) de l'auto-injecteur, ledit cylindre tubulaire (23) du piston (21) coulisse à l'intérieur desdits moyens de déclenchement (19) en entraînant le ressort (22), de sorte que le ressort (22) se comprime, jusqu'à ce que la partie de diamètre réduit (21a) du piston (21) coopère avec l'élément d'interaction (20) pour bloquer le piston (21) dans sa position armée.

5. Auto-injecteur selon la revendication 4, dans lequel lesdits moyens de déclenchement (19) sont réalisés sous la forme d'au moins une patte élastique et ledit élément d'interaction (20) est réalisé sous la forme d'un ergot disposé à l'extrémité libre de ladite patte élastique (19), ladite au moins une patte élastique (19) étant amenée dans sa position de libération de piston dès lors que ledit manchon de commande (17) est forcé dans sa position de déverrouillage et ladite au moins une patte élastique (19) étant amenée dans sa position de blocage du piston sous l'effet de la force exercée par ledit manchon de commande (17), dès lors que ledit élément d'interaction (20) coopère avec ladite partie de diamètre réduit (21a) dudit piston (21).

6. Auto-injecteur selon la revendication 4, dans lequel lesdits moyens de déclenchement (19) comprennent un anneau fendu (19) qui adopte sa position de libération du piston dès lors que le manchon de commande (17) est forcé dans sa position de déverrouillage et qui adopte sa position de blocage du piston dès lors qu'il coopère avec la partie de diamètre réduit (21a) dudit piston (21).

7. Auto-injecteur selon l'une quelconque des revendications précédentes, comportant une partie de couvercle (102) emboîtable sur la partie de boîtier (101), le dispositif d'injection automatique du produit contenu dans la seringue étant disposé dans la partie de couvercle (102), l'emboîtement de ladite partie de couvercle (102) sur ladite partie de boîtier (101) lors de sa fermeture amenant ledit piston (21) dans sa position armée et lesdits moyens de déclenchement (19) dans leur position de blocage.

8. Auto-injecteur selon la revendication 7, dans lequel ladite partie de couvercle (102) et ladite partie de boîtier (101) sont de forme générale cylindrique, ladite partie de couvercle (102) s'emboîtant axialement sur ladite partie de boîtier (101), ladite partie de boîtier (101) comportant lesdits moyens de réarmement qui comprennent ledit organe d'armement (150) coopérant avec ledit au moins un élément (125) solidaire du piston lors dudit emboîtement axial de ladite partie de couvercle (102) sur ladite partie de boîtier (101) pour amener ledit piston (21) dans sa position armée.

9. Auto-injecteur selon la revendication 8, dans lequel ledit organe d'armement (150) de la partie de boîtier (101) s'emmanche autour dudit manchon fixe (126), lors de l'emboîtement de la partie de couvercle (102) sur la partie de boîtier (101), en agissant sur ledit au moins un élément (125) solidaire du piston pour amener ledit piston (21) dans sa position armée.

10. Auto-injecteur selon la revendication 9, dans lequel ledit organe d'armement (150) est cylindrique et comporte au moins une fente axiale (156) pour permettre audit au moins un élément (125) solidaire du piston de coulisser par rapport audit organe d'armement (150) lorsque le piston (21) se déplace de sa position armée vers sa position de fin de course, ledit organe d'armement (150) pouvant être déplacé en rotation autour dudit manchon fixe (126) entre une position angulaire d'armement, où il coopère avec ledit au moins un élément (125) solidaire du piston pour armer ledit piston (21), et une position angulaire de libération, où ladite au moins une fente axiale (156) de l'organe d'armement (150) est disposée vis-à-vis dudit au moins un élément (125) solidaire du piston.

11. Auto-injecteur selon la revendication 10, dans lequel ladite partie de boîtier (101) comporte un tube (5) pouvant coulisser axialement par rapport audit organe d'armement (150) entre une position de repos où il recouvre l'aiguille (4) de la seringue (3) et une position d'actionnement où il agit comme organe d'actionnement du dispositif d'injection, son extrémité (5a) opposée à la seringue libérant les moyens de déclenchement (19) du dispositif d'injection, ledit tube (5) comportant un moyen de blocage (160) qui empêche tout déplacement axial du tube (5) sur ledit organe d'armement (150) lorsque celui-ci est dans sa position angulaire d'armement et qui permet ledit déplacement axial lorsque l'organe d'armement (150) est dans sa position angulaire de libération.

12. Auto-injecteur selon la revendication 11, dans lequel ledit moyen de blocage du tube (5) est réalisé sous la forme d'un doigt de blocage élastique (160) saillant à l'extérieur dudit tube (5), ledit tube (5) comportant en outre un doigt de butée (170) saillant à l'extérieur dudit tube (5) qui ne permet une séparation de la partie de boîtier (101) de la partie de couvercle (102) que lorsque l'organe d'armement (150) est dans sa position angulaire de libération.

13. Auto-injecteur selon la revendication 12, dans lequel la partie de couvercle (102) comporte une enveloppe externe cylindrique (103) qui s'emboîte autour du tube (5) de la partie de boîtier (101), le diamètre intérieur de ladite enveloppe externe (103) étant environ identique au diamètre extérieur dudit tube (5), de sorte que l'emboîtement de ladite enveloppe sur ledit tube force ledit doigt de blocage élastique (160) vers l'intérieur pour bloquer le déplacement axial du tube (5) par rapport audit organe d'armement (150), l'enveloppe (103) comportant à son extrémité ouverte une encoche d'introduction (165) pour introduire ladite patte de blocage (160) saillante, ladite encoche (165) étant disposée circonférentiellement de telle sorte que l'organe d'armement (150) est disposé dans sa position angulaire d'armement lors de l'emboîtement de la partie de couvercle (102) sur la partie de boîtier (101), l'enveloppe (103) comportant sur sa surface intérieure au moins une rainure axiale (175) s'étendant jusqu'à ladite extrémité ouverte, ladite au moins une rainure (175) étant décalée angulairement par rapport à ladite encoche (165) de telle sorte que ladite patte de blocage (160) pénètre dans ladite au moins une rainure axiale (175) lorsque l'organe d'armement (150) est dans sa position angulaire de libération.

14. Auto-injecteur selon la revendication 13, dans lequel ledit organe d'armement (150) comporte sur sa surface intérieure, à proximité de son extrémité qui coopère avec ledit au moins un élément (125) solidaire du piston, une rainure circonférentielle de solidarisation (155a) qui s'encliquette de manière amovible, après l'armement du piston (21), sur une nervure circonférentielle complémentaire (155b) prévue sur le manchon fixe (126), lesdites nervures et rainures de solidarisation (155a, b) assurant une solidarisation amovible de la partie de boîtier (101) avec la partie de couvercle (102) avant et après l'actionnement de l'auto-injecteur, et assurant une fixation inamovible de la partie de boîtier (101) sur la partie de couvercle (102) pendant l'actionnement de l'auto-injecteur, l'extrémité (5a) du tube (5) bloquant ladite rainure sur ladite nervure lorsque ledit tube (5) est dans sa position d'actionnement.

15. Auto-injecteur selon l'une quelconque des revendications précédentes, dans lequel est prévu un organe élastique sollicitant ladite seringue (3) légèrement hors de la partie de boîtier (101) de l'auto-injecteur lorsque la partie de couvercle (102) est ouverte, facilitant ainsi la préhension de ladite seringue.

## Patentansprüche

1. Nachladbarer Auto-Injektor, der einen Gehäuseteil (101) umfaßt, der dazu dient, eine Spritze (3) aufzunehmen, sowie einen Abdeckteil (102), wobei der Auto-Injektor eine automatische Injektionsvorrichtung für das in der Spritze enthaltene Produkt umfaßt, wobei diese Injektionsvorrichtung folgende Bestandteile aufweist:
- einen Kolben (21), der, in der vertikalen Position des Auto-Injektors, bei der sich die Spritze (3) unten befindet, einen oberen Teil (21b) und einen unteren Teil (21c) umfaßt, wobei der untere Teil (21c) mit dem Kolben der Spritze zusammenwirkt, wobei dieser Kolben (21) unter der Wirkung einer Betätigungsfeder (22) zwischen einer gespannten Position und einer Bewegungs-Endposition bewegbar ist, wobei die Feder (21) in der gespannten Position zusammengedrückt ist, und
- Auslöseeinrichtungen (19), die zwischen einer Blockierposition, in der sie den Kolben (21) in seiner gespannten Position halten, und einer Freigabeposition für den Kolben (21) bewegbar sind, wobei die Auslöseeinrichtungen (19) aus ihrer Blockierposition durch ein Betätigungsorgan freigegeben werden,
wobei der Auto-Injektor weiterhin Spanneinrichtungen für die automatische Injektionsvorrichtung umfaßt,
dadurch **gekennzeichnet**, daß die Spanneinrichtungen ein Spannorgan (150) umfassen, das mit wenigstens einem Element (125) zusammenwirkt, das während des Schließvorganges des Abdeckteils (102) mit dem Kolben (21) verbunden ist, um den Kolben (21) aus seiner Bewegungs-Endposition in seine gespannte Position zurückzubringen, wobei der Kolben (21) in einer festen Muffe (126) des Abdeckteils (102) gleitet, die wenigstens einen axialen Schlitz (127) aufweist, durch den hindurch das wenigstens eine mit dem Kolben verbundene Element (125) vorspringt, um mit dem Spannorgan (150) zusammenzuwirken.

2. Auto-Injektor nach Anspruch 1, bei dem eine Steuermuffe (17) vorgesehen ist, die zwischen einer Verriegelungsposition, in der sie die Auslöseeinrichtungen (19) in ihrer Blockierstellung und somit den Kolben (21) in seiner gespannten Position hält, und einer Entriegelungsstellung bewegbar ist, in der die Auslöseeinrichtungen (19) in ihre Freigabeposition für den Kolben (21) gelangen, wobei die Steuermuffe (17) in ihre Verriegelungsstellung durch eine Feder (18) vorgespannt ist und in ihre Entriegelungsstellung durch das Betätigungsorgan gedrückt wird.

3. Auto-Injektor nach Anspruch 2, bei dem die Auslöseeinrichtungen (19) elastisch sind und ein Wechselwirkungselement (20) umfassen, das an seiner Innenseite (20a) mit dem oberen Teil (21b) des Kolbens (21) und an seiner Außenseite (20b) mit der Steuermuffe (17) zusammenwirkt, wobei das Wechselwirkungselement (20) die Steuermuffe (17) in ihrer Entriegelungsstellung hält, wenn die elastischen Auslöseeinrichtungen (19) sich in ihrer Freigabeposition für den Kolben (21) befinden, wobei der obere Teil (21b) des Kolbens (21) einen Teil mit verringertem Durchmesser (21a) umfaßt, der mit dem Wechselwirkungselement (20) dann, wenn sich der Kolben (21) in seiner gespannten Position befindet, derart zusammenwirkt, daß die elastischen Auslöseeinrichtungen (19) ihre Blockierposition für den Kolben (21) annehmen, und gleichzeitig die Steuermuffe (17) freigeben, die ihre Verriegelungsposition einnimmt und dabei um das Wechselwirkungselement (20) der elastischen Auslöseeinrichtungen (19) herum in Eingriff tritt, und somit diese daran hindert, in ihre Freigabeposition für den Kolben (21) zurückzukehren, wodurch der Kolben (21) in seiner gespannten Position blockiert wird.

4. Auto-Injektor nach Anspruch 3, bei dem der obere Teil (21b) des Kolbens (21) einen hohlen Rohrzylinder (23) umfaßt, dessen äußere Oberfläche mit dem Wechselwirkungselement (20) der elastischen Auslöseeinrichtungen (19) zusammenwirkt, wobei der Rohrzylinder (23) an seinem bezüglich der Spritze proximalen Ende einen stumpfkegeligen Teil umfaßt, der den Teil mit vermindertem Durchmesser (21a) des Kolbens (21) bildet, wobei der Rohrzylinder (23) ein Ende der Betätigungsfeder (22) für den Kolben (21) aufnimmt, während das andere Ende der Feder (22) mit dem Gehäuse des Auto-Injektors derart fest verbunden ist, daß beim Öffnen und/oder Schließen des Abdeckteils (2, 102) des Auto-Injektors der Rohrzylinder (23) des Kolbens (21) im Inneren der Auslöseeinrichtungen (19) gleitet und dabei die Feder (22) derart antreibt, daß sich die Feder (22) zusammendrückt, bis der Teil (21a) mit vermindertem Durchmesser des Kolbens (21) mit dem Wechselwirkungselement (20) zusammenwirkt, um den Kolben (21) in seiner gespannten Position zu verriegeln.

5. Auto-Injektor nach Anspruch 4, bei dem die Auslöseeinrichtungen (19) in Form von wenigstens einer elastischen Klaue ausgebildet sind und das Wechselwirkungselement (20) in Form eines Zapfens ausgebildet ist, der am freien Ende der elastischen Klaue (19) angeordnet ist, wobei die wenigstens eine elastische Klaue (19) in ihre Freigabeposition für den Kolben gebracht wird, sobald die Steuermuffe (17) in ihre Entriegelungsstellung gedrückt wird, und wobei die wenigstens eine elastische Klaue (19) in ihre Blockierstellung für den Kolben unter dem Einfluß der Kraft gebracht wird, die von der Steuermuffe (17) ausgeübt wird, sobald das Wechselwirkungselement (20) mit dem Teil (21a) mit vermindertem Durchmesser des Kolbens (21) zusammenwirkt.

6. Auto-Injektor nach Anspruch 4, bei dem die Auslöseeinrichtungen (19) einen geschlitzten Ring (19) umfassen, der seine Freigabeposition für den Kolben einnimmt, sobald die Steuermuffe (17) in ihre Entriegelungsstellung gedrückt wird, und der seine Blockierstellung für den Kolben einnimmt, sobald er mit dem Teil (21a) mit vermindertem Durchmesser des Kolbens (21) zusammenwirkt.

7. Auto-Injektor nach einem der vorhergehenden Ansprüche, der einen auf den Gehäuseteil (101) aufsetzbaren Abdeckteil (102) umfaßt, wobei die automatische Injektionsvorrichtung für das in der Spritze enthaltene Produkt in dem Abdeckteil (102) angeordnet ist, wobei das Aufsetzen des Abdeckteils (102) auf den Gehäuseteil (101) während seines Schließens den Kolben (21) in seine gespannte Position und die Auslöseeinrichtungen (19) in ihre Blockierposition bringt.

8. Auto-Injektor nach Anspruch 7, bei dem der Abdeckteil (102) und der Gehäuseteil (101) im wesentlichen eine zylindrische Form besitzen, wobei der Abdeckteil (102) axial auf den Gehäuseteil (101) aufsetzbar ist, wobei der Gehäuseteil (101) die Spanneinrichtungen umfaßt, welche das Spannorgan (150) umfassen, das mit dem wenigstens einen Element (125), das fest mit dem Kolben verbunden ist, während des axialen Aufsetzens des Abdeckteils (102) auf den Gehäuseteil (101) zusammenwirkt, um den Kolben (21) in seine gespannte Position zu bringen.

9. Auto-Injektor nach Anspruch 8, bei dem sich das Spannorgan (150) des Gehäuseteils (101) um die feste Muffe (126) während des Aufsetzens des Abdeckteils (102) auf den Gehäuseteil (101) aufdrückt und dabei auf das wenigstens eine Element (125) einwirkt, das mit dem Kolben verbunden ist, um den Kolben (21) in seine gespannte Position zu bringen.

10. Auto-Injektor nach Anspruch 9, bei dem das Spannorgan (150) zylindrisch ist und wenigstens einen axialen Schlitz (156) umfaßt, um es dem wenigstens einen, mit dem Kolben verbundenen Element (125) zu ermöglichen, bezüglich des Spannorgans (150) zu gleiten, wenn sich der Kolben (21) aus seiner gespannten Position zu seiner Bewegungs-Endposition bewegt, wobei das Spannorgan (150) in drehender Weise um die feste Muffe (126) zwischen einer Spann-Winkelposition, in der es mit dem wenigstens einen, mit dem Kolben verbundenen Element (125) zusammenwirkt, um den Kolben zu spannen, und einer Winkel-Freigabeposition verschiebbar ist, in welcher der wenigstens eine axiale Schlitz (156) des Spannorgans (150) gegenüber dem wenigstens einen fest mit dem Kolben verbundenen Element (125) angeordnet ist.

11. Auto-Injektor nach Anspruch 10, bei dem der Gehäuseteil (101) ein Rohr (5) umfaßt, das axial bezüglich des Spannorgans (150) zwischen einer Ruhelage, in der es die Nadel (4) der Spritze (3) abdeckt, und einer Betätigungsposition gleiten kann, in der es als Betätigungsorgan für die Injektionsvorrichtung arbeitet, wobei sein der Spritze gegenüberliegendes Ende (5a) die Auslöseeinrichtungen (19) der Injektionsvorrichtung freigibt, wobei das Rohr (5) eine Blockiereinrichtung (160) umfaßt, die jegliche axiale Verschiebung des Rohrs (5) auf dem Spannorgan (150) verhindert, wenn sich dieses in seiner Spann-Winkelposition befindet, und das die axiale Verschiebung ermöglicht, wenn sich das Spannorgan (150) in seiner Freigabe-Winkelposition befindet.

12. Auto-Injektor nach Anspruch 11, bei dem die Blockiereinrichtung für das Rohr (5) in Form eines elastischen Blockierfingers (160) ausgebildet ist, der zur Außenseite des Rohrs (5) hin vorspringt, wobei das Rohr (5) im übrigen einen Anschlagfinger (170) umfaßt, der zur Außenseite des Rohrs (5) hin vorspringt und der nur dann eine Trennung des Gehäuseteils (101) vom Abdeckteil (102) ermöglicht, wenn das Spannorgan (150) sich in seiner Freigabe-Winkelposition befindet.

13. Auto-Injektor nach Anspruch 12, bei dem der Abdeckteil (102) eine äußere zylindrische Hülle (103) umfaßt, die auf das Rohr (5) des Gehäuseteils (101) aufbringbar ist, wobei der Innendurchmesser der äußeren Hülle (103) ungefähr identisch mit dem Außendurchmesser des Rohrs (5) derart ist, daß das Aufbringen der Hülle auf das Rohr den elastischen Blockierfinger (160) zum Inneren hin drückt, um die axiale Verschiebung des Rohrs (5) bezüglich des Spannorgans (150) zu blockieren, wobei die Hülle (103) an ihrem offenen Ende eine Einführkerbe (165) umfaßt, um die vorspringende Blockierklaue (160) einzuführen, wobei die Kerbe (165) umfangsmäßig derart angeordnet ist, daß das Spannorgan (150) während des Aufbringens des Abdeckteils (102) auf den Gehäuseteil (101) in seiner Spann-Winkelposition angeordnet ist, wobei die Hülle (103) auf ihrer inneren Oberfläche wenigstens eine axiale Rille (175) umfaßt, die sich bis zum offenen Ende erstreckt, wobei die wenigstens eine Rille (175) winkelmäßig bezüglich der Kerbe (165) derart versetzt ist, daß die Blockierklaue (160) in die wenigstens eine axiale Rille (175) eindringt, wenn sich das Spannorgan (150) in seiner Freigabe-Winkelposition befindet.

14. Auto-Injektor nach Anspruch 13, bei dem das Spannorgan (150) auf seiner inneren Oberfläche in der Nähe seines Endes, das mit dem wenigstens einen mit dem Kolben verbundenen Element (125) zusammenwirkt, eine in Umfangsrichtung verlaufende Verbindungsrille (155a) umfaßt, die nach dem Spannen des Kolbens (21) in lösbarer Weise auf einer komplementären, umfangsmäßig verlaufenden Rippe (155b) einrastet, die auf der festen Muffe (126) vorgesehen ist, wobei die Verbindungsrippe und Verbindungsrille (155a, b) eine lösbare Verbindung des Gehäuseteils (101) mit dem Abdeckteil (102) vor und nach der Betätigung des Auto-Injektors sicherstellen und eine nichtlösbare Befestigung des Gehäuseteils (101) auf dem Abdeckteil (102) während der Betätigung des Auto-Injektors sicherstellen, da das Ende (5a) des Rohrs (5) die Rille auf der Rippe blockiert, wenn sich das Rohr (5) in seiner Arbeitsposition befindet.

15. Auto-Injektor nach einem der vorhergehenden Ansprüche, bei dem ein elastisches Organ vorgesehen ist, das die Spritze (3) geringfügig aus dem Gehäuseteil (101) des Auto-Injektors heraus vospannt, wenn der Abdeckteil (102) offen ist, wodurch das Ergreifen der Spritze erleichtert wird.

## Claims

1. A reloadable auto-injector including a housing portion (101) designed to receive a syringe (3), and a cover portion (102), said auto-injector incorporating an injection device for automatically injecting the substance contained in the syringe, said injection device comprising:
a piston (21) comprising, in the vertical position of the auto-injector with the syringe (3) at the bottom, a high portion (21b) and a low portion (21c), said low portion (21c) co-operating with the plunger of the syringe, said piston (21) being mounted to move, under the effect of an actuating spring (22) between a cocked position and an end-of-stroke position, said spring (22) being compressed in said cocked position; and
trigger means (19) mounted to move between a blocking position in which they hold the piston (21) in its cocked position, and a releasing position in which they release the piston (21), said trigger means (19) being released from their blocking position by an actuating member;
the auto-injector further including recocking means for recocking the automatic injection device, said auto-injector being characterized in that said recocking means include a cocking member (150) that co-operates with at least one element (125) secured to the piston (21) during the operation of closing said cover portion (102) in order to bring said piston (21) from its end-of-stroke position to its cocked position, said piston (21) sliding inside a fixed sleeve (126) in the cover portion (102) which includes at least one axial slot (127) through which said at least one element (125) secured to the piston projects, in order to cooperate with said cocking member (150).

2. An auto-injector according to claim 1, in which a control sleeve (17) is provided mounted to move between a locking position in which it holds the trigger means (19) in their blocking position and thus the piston (21) in its cocked position and an unlocking position in which said trigger means (19) come into their releasing position in which they release the piston (21), said control sleeve (17) being urged towards its locking position by a spring (18), and being forced into its unlocking position by said actuating member.

3. An auto-injector according to claim 2, in which said trigger means (19) are resilient, and they are provided with an interaction element (20) co-operating on its inside (20a) with the high portion (21b) of the piston (21), and on its outside (20b) with the control sleeves (17), said interaction element (20) holding said control sleeve (17) in the unlocking position when said resilient trigger means (19) are in their releasing position in which they release the piston (21), said high portion (21b) of the piston (21) including a smaller-diameter portion (21a) which co-operates with said interaction element (20) when the piston (21) is in its cocked position, so that said resilient trigger means (19) take up their blocking position in which they block said piston (21), thereby simultaneously releasing said control sleeve (17) which takes up its locking position by coming into engagement around said interaction element (20) of said resilient trigger means (19), thereby preventing said trigger means from returning to their releasing position in which the release the piston (21), the piston (21) then being blocked in its cocked position.

4. An auto-injector according to claim 3, in which the high portion (21b) of the piston (21) comprises a hollow tubular cylinder (23) whose outside surface co-operates with said interaction element (20) of said resilient trigger means (19), that end of said tubular cylinder (23) which is closer to the syringe being provided with a frustoconical portion forming the smaller-diameter portion (21a) of the piston (21), said tubular cylinder (23) receiving an end of said spring (22) for actuating the piston (21), the other end of said spring (22) being secured to the housing of the auto-injector, so that, on opening and/or on closing the cover portion (2, 102) of the auto-injector, said tubular cylinder (23) of the piston (21) slides inside said trigger means (19), thereby entraining the spring (22) so that it is compressed, until the smaller-diameter portion (21a) of the piston (21) co-operates with the interaction element (20) to block the piston (21) in its cocked position.

5. An auto-injector according to claim 4, in which said trigger means (19) are implemented in the form of at least one resilient tab, and said interaction element (20) is implemented in the form of a lug disposed at the free end of said resilient tab (19), said at least one resilient tab (19) being brought into its piston-releasing position whenever said control sleeve (17) is forced into its unlocking position, and said at least one resilient tab (19) being brought into its piston-blocking position under the effect of the force exerted by said control sleeve (17), whenever said interaction element (20) co-operates with said smaller-diameter portion (21a) of said piston (21).

6. An auto-injector according to claim 4, in which said trigger means (19) comprise a split ring (19) which takes up its piston-releasing position whenever the control sleeve (17) is forced into its unlocking position, and which takes up its piston-blocking position whenever it co-operates with the smaller-diameter portion (21a) of said piston (21).

7. An auto-injector according to any preceding claim, including a cover portion (102) that can be interfitted with the housing portion (101) by being fitted thereon, the injection device for automatically injecting the substance contained in the syringe being disposed in the cover portion (102), said cover portion (102) with said housing portion (101) on being closed bringing said piston (21) into its cocked position and brining said trigger means (19) into their blocking position.

8. An auto-injector according to claim 7, in which said cover portion (102), and said housing portion (101) are generally cylindrical in shape, said cover portion (102) interfitting axially with said housing portion (101), said housing portion (101) including said recocking means which comprise said cocking member (150) that co-operates with said at least one element (125) secured to the piston on axially fitting said cover portion (102) onto said housing portion (101) so as to bring said piston (21) into its cocked position.

9. An auto-injector according to claim 8, in which said cocking member (150) in the housing portion (101) comes into engagement around said fixed sleeve (126) on fitting the cover portion (102) onto the housing portion (101), thereby acting on said at least one element (125) secured to the piston to bring said piston (21) into its cocked position.

10. An auto-injector according to claim 9, in which said cocking member (150) is cylindrical and is provided with at least one axial slot (156) to enable said at least one element (125) secured to the piston to slide relative to said cocking member (150) while the piston (21) is being displaced from its cocked position to its end-of-stroke position, it being possible for said cocking member (150) to be rotated about said fixed sleeve (126) between a cocking angular position, in which it co-operates with said at least one element (125) secured to the piston to cock said piston (21), and a releasing angular position, in which said at least one axial slot (156) in the cocking member (150) is disposed facing said at least one element (125) secured to the piston.

11. An auto-injector according to claim 10, in which said housing portion (101) includes a tube (5) mounted to slide axially relative to said cocking member (150) between a rest position in which it covers up the needle (4) of the syringe (3) and an actuating position in which it acts as an actuating member for actuating the injection device, its end (5a) further from the syringe releasing the trigger means (19) for triggering the injection means, said tube (5) being provided with blocking means (160) which prevent the tube (5) from being displaced axially on said cocking member (150) when said cocking member is in its cocking angular position, and which enable said tube to be displaced axially thereon, when the cocking member (150) is in its releasing angular position.

12. An auto-injector according to claim 11, in which said blocking means on the tube (5) are implemented in the form of a resilient blocking finger (160) projecting outwards from said tube (5), said tube (5) further including an abutment finger (170) projecting outwards from said tube (5), which finger allows the housing portion (101) to be separated from the cover portion (102) only when the cocking member (150) is in its releasing angular position.

13. An auto-injector according to claim 12, in which the cover portion (102) includes a cylindrical outer casing (103) which interfits with the tube (5) of the housing portion (101) by fitting around said tube, the inside diameter of said outer casing (103) being approximately identical to the outside diameter of said tube (5), so that said casing being interfitted with said tube forces said resilient blocking finger (160) inwards so as to prevent the tube (5) from being displaced axially relative to said cocking member (150), the open end of the casing (103) being provided with an insertion notch (165) for inserting said projecting blocking tab (160), said notch (165) being disposed circumferentially such that the cocking member (150) is disposed in its cocking angular position when the cover portion (102) is interfitted with the housing portion (101), the inside surface of the casing (103) being provided with at least one axial groove (175) extending to said open end, said at least one groove (175) being offset angularly relative to said notch (165) so that said blocking tab (160) penetrates into said at least one axial groove (175) when said cocking member (150) is in its releasing angular position.

14. An auto-injector according to claim 13, in which the inside surface of said cocking member (150) in the vicinity of the end thereof that co-operates with said at least one element (125) secured to the piston is provided with a securing circumferential groove (155a) which is releasably snap-fastened, after the piston (21) has been cocked, on a complementary circumferential rib (155b) provided on the fixed sleeve (126), said securing rib and groove (155a, b) securing the housing portion (101) to the cover portion (102) in releasable manner before and after the auto-injector is actuated, and fixing the housing portion (101) to the cover portion (102) in non-releasable manner while the auto-injector is being actuated, the end (5a) of the tube (5) blocking said groove on said rib when said tube (5) is in its actuating position.

15. An auto-injector according to any preceding claim, in which a resilient member is provided urging said syringe (3) slightly proud of the housing portion (101) of the auto-injector when the cover portion (102) is open, thereby making said syringe easier to grasp.
